(19) **Europäisches Patentamt / European Patent Office / Office européen des brevets**

(11) **EP 4 620 397 A1**

(12) **EUROPEAN PATENT APPLICATION**

(43) Date of publication:
24.09.2025 Bulletin 2025/39

(51) International Patent Classification (IPC):
$A61B\ 6/00^{(2024.01)}$   $A61B\ 6/58^{(2024.01)}$

(21) Application number: 25165393.7

(22) Date of filing: 21.03.2025

(52) Cooperative Patent Classification (CPC):
A61B 6/542; A61B 6/487; A61B 6/5258;
A61B 6/545; A61B 6/583

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 21.03.2024 US 202418612731
14.02.2025 JP 2025022470

(71) Applicant: Canon Medical Systems Corporation
Tochigi 324-0036 (JP)

(72) Inventors:
• HU, Yi
  Otawara-shi, 324-0036 (JP)
• LI, Shijie
  Otawara-shi, 324-0036 (JP)
• MANAK, Joseph
  Otawara-shi, 324-0036 (JP)
• SHARMA, Shobhit
  Otawara-shi, 324-0036 (JP)
• BAUMGART, John
  Otawara-shi (JP)

(74) Representative: Marks & Clerk LLP
15 Fetter Lane
London EC4A 1BW (GB)

(54) **X-RAY DIAGNOSTIC APPARATUS AND METHOD**

(57) An X-ray diagnostic apparatus (100) of an embodiment includes a setting unit (212) that inputs an X-ray image acquired using a first set of exposure parameters into a first model to obtain a second set of exposure parameters, an acquisition unit (211) that inputs the X-ray image acquired using the second set of exposure parameters into a second model trained together with the first model to obtain a restored image, and a control unit (213) that outputs the restored image.

FIG.1

**Description**

<u>FIELD</u>

Embodiments described herein relate generally to an X-ray diagnostic apparatus and method.

<u>BACKGROUND</u>

The background description provided herein is for the purpose of generally presenting the context of the disclosure. Work of the presently named inventors, to the extent the work is described in this background section, as well as aspects of the description that may not otherwise qualify as prior art at the time of filing, are neither expressly nor impliedly admitted as prior art against the present disclosure.

The image quality of fluoroscopy sequences can depend on both exposure conditions and image restoration processes. In some exposure control processes, the acquisition or exposure parameters of one frame (e.g., mA, ms, kV, filters, etc.) can be determined based on the contents (e.g., a histogram) of a current frame to maximize the image quality (e.g., contrast-to-noise ratio). However, the exposure dose can be set generally low during fluoroscopy procedures, which can present challenges for the downstream image denoising or image restoration processes. Thus, a method providing varying, customized acquisition parameters during fluoroscopy procedures prior to the image denoising and image restoration processes is desired.

<u>Summary of Invention</u>

An X-ray diagnosis apparatus provided according to one aspect of the present invention includes: a setting unit that inputs an X-ray image acquired using a first set of exposure parameters into a first model to obtain a second set of exposure parameters; an acquisition unit that inputs the X-ray image acquired using the second set of exposure parameters into a second model trained together with the first model to obtain a restored image; and a control unit (213) that outputs the restored image.

The setting unit may input a plurality of X-ray images acquired using the first set of exposure parameters into the first model to obtain the second set of exposure parameters.

The setting unit may input a plurality of adjacent frames of X-ray images acquired using the first set of exposure parameters into the first model to obtain the second set of exposure parameters.

In the training process, the first model may be trained to receive a first training image and output a set of training exposure parameters, the second model may be trained to receive a second training image acquired based on the set of training exposure parameters and output a training restored image, and the first model may be updated based on a comprehensive evaluation of an X-ray radiation dose corresponding to the set of training exposure parameters and image quality of the training restored image.

The setting unit may obtain a sequence of a plurality of X-ray images including a first X-ray image and a second X-ray image, the first and second X-ray images being acquired sequentially using the first set of exposure parameters of an image scanning apparatus, input the obtained first and second X-ray images into the trained first model to obtain the second set of exposure parameters, which is output from the first model, the acquisition unit may obtain a third X-ray image that was acquired by the image scanning apparatus using the second set of exposure parameters, input at least the second X-ray image and the third X-ray image into the trained second model to obtain a restored third X-ray image as the restored image, which is output from the second model, and the control unit may output the restored third X-ray image as the restored image, the first model and the second model may be trained together using a training sequence of X-ray images in a training process, and during the training process, inputs to the second model may be outputs of the first model and the outputs of second model may be a sequence of restored X-ray images.

In the training process, the first model may be trained using a first loss function having a first term representing a value relating to total dosage used in acquiring the training sequence of X-ray images and a second term representing a value relating to total error in the sequence of restored X-ray images, and the second model may be trained using a second loss function having a term representing the total error in the sequence of restored X-ray images.

The first set of exposure parameters in the exposure parameters may include a first dose level, the second set of exposure parameters in the exposure parameters may include a second dose level, and the first dose level may be lower than the second dose level.

The first model and the second model may be neural network models.

The first model and the second model may alternatively be trained during the training process.

An X-ray diagnosis apparatus provided according to one aspect of the present invention includes processing unit configured to perform a training process by obtaining a sequence of training X-ray images sequentially acquired using a corresponding set of exposure parameters of an image scanning apparatus, inputting a pair of X-ray images of the sequence of training X-ray images into a first model to obtain an output set of exposure parameters, which is output from the first model, generating a simulation image based on the output set of exposure parameters, inputting the simulation image into a second model to obtain a restored X-ray image, which is output from the second model, comparing the restored X-ray image to a corresponding target X-ray image, of an obtained sequence of target X-ray images, to generate an image error term, repeating the inputting, generating, inputting, and comparing steps for sequential pairs of the obtained sequence of training X-ray images, and updating one of the first model and the second model.

The processing unit may further perform the training process by updating the first model using a first loss function having a first term representing a value relating to total dosage used in acquiring the training sequence of X-ray images and a second term representing a total of the image error terms for the sequence of restored X-ray images, and updating the second model using a second loss function having a term representing the total of the image error terms for the sequence of restored X-ray images.

In the updating, the processing unit may further be configured to: update the first model using the first loss function while keeping the second model fixed, and update the second model using the second loss function while keeping the first model fixed.

In the updating, the processing unit may further repeat the training process for another sequence of training X-ray images sequentially acquired using another corresponding set of exposure parameters and another sequence of target X-ray images.

The step of inputting the generated simulation image into the second model may further comprise inputting another simulation image into the second model together with the simulation image, the another simulation image may be generated based on another set of exposure parameters.

The first model and the second model may be neural network models.

The sequence of training X-ray images may be clinical X-ray images or X-ray images obtaining from imaging a phantom.

In the generating, the processing unit may further be configured to generate a projection without any degradation based on a digital phantom, and generate the simulated X-ray image from the generated projection based on the output set of exposure parameters.

An method comprising: inputting an X-ray image acquired using a first set of exposure parameters into a first model to obtain a second set of exposure parameters; inputting the X-ray image acquired using the second set of exposure parameters into a second model trained together with the first model to obtain a restored image; and outputting the restored image.

An method, for performing a training process, comprising: obtaining a sequence of training X-ray images sequentially acquired using a corresponding set of exposure parameters of an image scanning apparatus; inputting a pair of X-ray images of the sequence of training X-ray images into a first model to obtain an output set of exposure parameters, which is output from the first model; generating a simulation image based on the output set of exposure parameters; inputting the simulation image into a second model to obtain a restored X-ray image, which is output from the second model, comparing the restored X-ray image to a corresponding target X-ray image, of an obtained sequence of target X-ray images, to generate an image error term; and repeating the inputting, generating, inputting, and comparing steps for sequential pairs of the obtained sequence of training X-ray images, and updating one of the first model and the second model.

BRIEF DESCRIPTION OF THE DRAWINGS

FIG. 1 is a schematic describing the acquisition parameter adjustment process, according to an embodiment of the present disclosure;
FIG. 2 is a schematic describing the acquisition parameter adjustment process via frame rate adjustment, according to an embodiment of the present disclosure;
FIG. 3 is a schematic of an image restoration process, according to an embodiment of the present disclosure;
FIG. 4 is a schematic describing a neural network architecture for image restoration, according to an embodiment of the present disclosure;
FIG. 5 is a schematic describing the neural network training, according to an embodiment of the present disclosure;
FIG. 6A and FIG. 6B are schematics describing training dataset generation, according to an embodiment of the present disclosure;
FIG. 7A and FIG. 7B show a non-limiting example of a flow chart for a method of acquisition parameter adjustment, according to an embodiment of the present disclosure;
FIG. 7C shows a non-limiting example of a flow chart for a method of acquisition parameter adjustment, according to an embodiment of the present disclosure;

FIG. 7D shows a non-limiting example of a flow chart for a method of acquisition parameter adjustment, according to an embodiment of the present disclosure;

FIG. 8A shows an example of a general artificial neural network (ANN) having N inputs, K hidden layers, and three outputs, according to an embodiment of the present disclosure;

FIG. 8B shows a non-limiting example of a convolutional neural network (CNN), as in the present disclosure;

FIG. 9 is a block diagram of an X-ray apparatus, according to an embodiment of the present disclosure;

FIG. 10 is a schematic of a hardware system for performing a method, according to an embodiment of the present disclosure; and

FIG. 11 is a schematic of a hardware configuration of a device for performing a method, according to an embodiment of the present disclosure.

## DETAILED DESCRIPTION

The following disclosure provides many different embodiments, or examples, for implementing different features of the provided subject matter. Specific examples of components and arrangements are described below to simplify the present disclosure. These are, of course, merely examples and are not intended to be limiting. For example, the formation of a first feature over or on a second feature in the description that follows may include embodiments in which the first and second features are formed in direct contact, and may also include embodiments in which additional features may be formed between the first and second features, such that the first and second features may not be in direct contact. In addition, the present disclosure may repeat reference numerals and/or letters in the various examples. This repetition is for the purpose of simplicity and clarity and does not in itself dictate a relationship between the various embodiments and/or configurations discussed. Further, spatially relative terms, such as "top," "bottom," "beneath," "below," "lower," "above," "upper" and the like, may be used herein for ease of description to describe one element or feature's relationship to another element(s) or feature(s) as illustrated in the figures. The spatially relative terms are intended to encompass different orientations of the device in use or operation in addition to the orientation depicted in the figures. The apparatus may be otherwise oriented (rotated 90 degrees or at other orientations) and the spatially relative descriptors used herein may likewise be interpreted accordingly.

The order of discussion of the different steps as described herein has been presented for clarity sake. In general, these steps can be performed in any suitable order. Additionally, although each of the different features, techniques, configurations, etc., herein may be discussed in different places of this disclosure, it is intended that each of the concepts can be executed independently of each other or in combination with each other. Accordingly, the present embodiment can be embodied and viewed in many different ways.

X-ray imaging systems and methods are widely used medical imaging tools for diagnosis and clinical interventions. Radiography systems are essential tools in interventional radiology procedures which may vary from a few minutes to several hours. Radiography systems generally create two-dimensional projection images through a subject's body. Radiography systems generally create two-dimensional projection images through a subject's body. A radiation source, such as an X-ray tube, irradiates the body from one side. A collimator, generally adjacent to the X-ray source, limits the angular extent of the X-ray beam, so that radiation impinging on the body is substantially confined to a cone-beam/fan-beam region (i.e., an X-ray projection volume) defining an image volume of the body. At least one detector on the opposite side of the body receives radiation transmitted through the body substantially in the projection volume. The attenuation of the radiation that has passed through the body is measured by processing electrical signals received from the detector.

In both fluoroscopic mode and acquisition mode, a sequence of X-ray exposures can be collected at a selected frame rate with a dose, tube voltage, scan time, etc. In clinical practice, an object of interest in the image for a radiologist or operator can vary frequently (for example from a guide wire, to a catheter, iodinated blood vessel, or a stent) during the interventional procedure, and exposure parameters can be adjusted to better image the object of interest. Notably, during time periods where there is not as much movement or change for the object of interest, the exposure parameters can be adjusted to reduce a dose or radiation exposure level to a patient.

Some fluoroscopy systems can implement an image restoration process after an image is obtained by the system. In some fluoroscopy systems, noise reduction and contrast increase can be desired targets for automatic exposure determination and adjustment. Some automatic exposure control processes can control the fluoroscopy system in a way to maximize the output image quality from a physics perspective.

Image restoration processes can depend on prior information. For example, image sequences can provide prior information that can be used for image restoration. Notably, exposure control can depend on a histogram of an image (in the image sequence) and can be handled separately from the later image restoration process. This method of handling exposure control does not necessarily use the prior information.

Thus, in the present embodiment, a system and method for acquisition parameter adjustment that can control the acquisition parameters via the image restoration process are described.

In one embodiment, a neural network can be used to identify informative frames (or informative scenarios, or informative images). As described in the present embodiment, an informative frame can be a frame or image including a change or difference as compared to a previous frame (or frames). For example, an informative frame can be a frame including an object, such as a stent, being deployed where a previous frame did not include the stent or the stent position had not changed beyond a threshold parameter. For example, an informative frame can be a frame including vessels where a contrast agent has been injected where a previous frame did not include the injected contrast agent. For example, an informative frame can be a frame including an object of interest changing characteristics or parameters quickly or often, such as regions including blood flow, a heartbeat, etc. In general, informative frames are frames or images where a noticeable event or changes are occurring. Therefore, a more accurate or less noisy acquisition of the informative frames, and the frames subsequent to the informative frames, can be desired.

In one embodiment, upon determining a frame is an informative frame, exposure parameters can be adjusted based on the informative frame. The exposure parameters can include, but are not limited to, a dose, an acquisition frame rate, an acquisition time, a voltage, and a filter. The exposure parameters can be used to obtain the informative frames having more optimized exposure parameters.

In one embodiment, a neural network can be used to analyze the informative frames and corresponding exposure parameters as training data. Notably, simulated images and clinical images can be used as the training data.

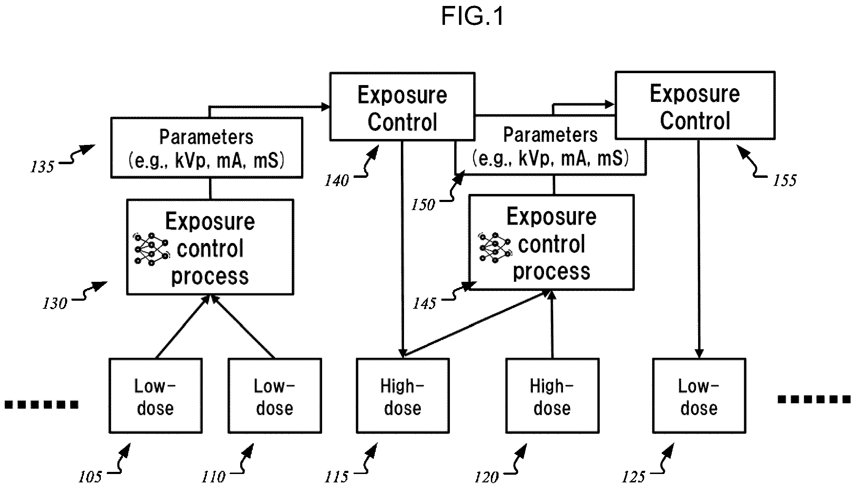

To this end, FIG. 1 is a schematic describing the acquisition parameter adjustment process, according to an embodiment of the present disclosure. In one embodiment, a first image 105 can be obtained via a fluoroscopy system with a first exposure setting or exposure parameters. The fluoroscopy system can include, for example, an X-ray source configured to emit X-ray radiation at a predetermined intensity, and processing circuitry, such as a computer, to analyze any images obtained by the fluoroscopy system. Notably, the first image 105 can be a low-dose image, as shown. The low dose of the first image 105 can be understood to mean that the first image 105 was obtained using an exposure parameter that includes a low dose setting or low radiation level for the X-ray source (or other radiation source in other imaging systems). The first image 105 can be analyzed by the processing circuitry (the fluoroscopy system) to identify any object of interest disposed in the first image 105, for example a stent. In the first image 105, the processing circuitry can determine first parameters of the object of interest, such as a first location of the object of interest.

In one embodiment, a second image 110 can be obtained via the fluoroscopy system with the first exposure setting. The second image 110 can also be a low-dose image, as shown. The second image 110 can be analyzed by the processing circuitry to identify the object of interest disposed in the second image 110 and second parameters of the object of interest, such as a second location of the object of interest. For example, the object of interest can move between acquisitions and the second location of the object of interest is not the same as the first location. The processing circuitry can determine a difference between the first parameters and the second parameters, such as the first location and the second location. The difference between the first location and the second location can be described as a change in distance. The difference between the first location and the second location can be used to determine whether the first image 105 and the second image 110 are informative frames.

In one embodiment, a first exposure control process 130 performed by the processing circuitry (the fluoroscopy system) can analyze the first image 105 and the second image 110 to determine whether the first image 105 and the second image 110 are informative frames. Again, the object of interest (or multiple objects of interest) can be determined in the first image 105 and the second image 110, as well as the corresponding first parameters and the corresponding second parameters (e.g., the corresponding first location and the corresponding second location). Upon determining the difference between the first location of the object of interest in the first image 105 and the second location of the object of interest in the second image 110 is greater than a predetermined threshold (or certain threshold or set threshold or target threshold), the exposure control process can determine that the first image 105 and the second image 110 are informative frames. That is, the first image 105 and the second image 110 include a change in the object of interest that can be imaged at a higher dose or exposure in order to obtain an image with less noise compared to the same image obtained at a lower dose.

In one embodiment, denoising need not be the target goal of increasing the exposure. For example, the informative frames can inform something other than denoising. For example, general tracking of the object of interest can be the target goal. For example, higher dose images can localize the object of interest that can be tracked later in a lower dose image. Higher dose image acquisitions can thus establish parameters of the object of interest, such as a shape, size, etc. of the object of interest being tracked through lower dose image acquisitions without having to have the future low dose image acquisitions be as high dose while still tracking the object of interest effectively or more accurately compared to a process using only low dose image acquisitions.

In one embodiment, the first parameters (and the second parameters and the parameters of the object of interest in subsequent acquisitions) can be, for example, a geometry of the object of interest, a size of the object of interest, a density of the object of interest, or any other measurable characteristics of the object of interest as the object of interest appears in the image or is represented in the imaging (projection) data. In one example, the object of interest is an artery with blood flow, and a changing density (or corresponding changing brightness/contrast) of the blood flow can signal that the frames or images capturing the blood flow are informative frames.

In one embodiment, upon determining that the first image 105 and the second image 110 are informative frames, the fluoroscopy system can adjust first exposure parameters 135 via a first exposure control 140. The first exposure parameters 135 can be adjusted based on the informative frames, such as the exposure parameters of the informative frames, the object of interest in the informative frames, and determined image parameters of the informative frames (brightness, contrast, etc.). The first exposure control 140 can be configured to adjust, for example, the dose, voltage, frame rate, filter, etc. in the first exposure parameters 135. As shown in FIG. 1, the first exposure control 140 can increase the dose, followed by acquisition of a third image 115. Thus, the third image 115 can be labeled as a high-dose image. Similarly, a fourth image 120 can be obtained, also at the high dose.

In one embodiment, upon determining the first image 105 and the second image 110 are not informative frames, the fluoroscopy system can maintain the current first exposure parameters 135 used to obtain the subsequent images, which can continue to be considered low-dose images. For example, the fluoroscopy system can determine that the difference between the first location of the object of interest in the first image 105 and the second location of the object of interest in the second image 110 is not greater than the predetermined threshold, and therefore maintain the low dose used to obtain the images.

In one embodiment, the processing circuitry can analyze the second image 110 and the third image 115 to determine whether the second image 110 and the third image 115 are informative frames. Again, the object of interest (or multiple objects of interest) can be determined in the second image 110 and the third image 115, and the corresponding second location for the object of interest in the second image 110 and a corresponding third location for the object of interest in the third image 115 can be determined. Upon determining that the difference between the second location of the object of interest in the second image 110 and the third location of the object of interest in the third image 115 is still greater than the predetermined threshold, the high dose can be maintained.

In one embodiment, a second exposure control process 145 performed by the processing circuitry can analyze the third image 115 and the fourth image 120 to determine whether the third image 115 and the fourth image 120 are informative frames. Again, the object of interest (or multiple objects of interest) can be determined in the third image 115 and the fourth image 120, and the corresponding third location for the object of interest in the third image 115 and a corresponding fourth location for the object of interest in the fourth image 120. Upon determining that the difference between the third location of the object of interest in the third image 115 and the fourth location of the object of interest in the fourth image 120 is not greater than the predetermined threshold, the exposure control process can determine that the third image 115 and the fourth image 120 are not informative frames. That is, it is possible to determine that the third image 115 and the fourth image 120 have negligible changes or no changes in the object of interest. For example, this can occur in a clinical setting where the patient is being imaged during a medical procedure, but the medical professionals are not currently adjusting the object of interest. For example, a surgeon can guide a stent into the imaging area and leave the stent in place momentarily. Thus, the high dose imaging can be used while guiding the stent into place, but the patient need not be exposed to the high dose once the stent is (temporarily or permanently) arranged because the imaging of the object of interest at a high resolution or low noise quality is not needed at the current time.

In one embodiment, upon determining the third image 115 and the fourth image 120 are not informative frames, the fluoroscopy system can adjust second exposure parameters 150 via a second exposure control 155. The second exposure parameters 150 can be adjusted back to a low dose, or a dose lower than a dose used to obtain the third image 115 and the fourth image 120 that were considered high-dose images. The second exposure control 155 can be configured to adjust, for example, the dose, voltage, imaging time, frame rate, filter, etc. in the second exposure parameters 150. As shown in FIG. 1, the second exposure control 155 can decrease the dose, followed by acquisition of a fifth image 125. Thus, the fifth image 125 can be labeled as a low-dose image.

In one embodiment, the first exposure control 140 and the second exposure control 155 can be an application or module included in the fluoroscopy system configured to receive data regarding the informative frames and/or data regarding the exposure parameters used to obtain the informative frames, and adjust the fluoroscopy system. For example, the first exposure control 140 and the second exposure control 155 can be configured to adjust the exposure parameters described previously, such as the dose, voltage, frame rate, filter, etc. The first exposure control 140 and the second exposure control 155 can be configured to adjust the exposure parameters by a processor, such as an ASIC, FPGA, etc. In one embodiment, the first exposure control 140 and the second exposure control 155 can be configured to adjust the exposure parameters via manual input from a user, such as technician. For example, the technician can continually monitor generated images and adjust the exposure parameters using an input device, such as a software program configured to adjust the exposure parameters, a physical feature (e.g., knob, slider, button, switch, etc.) on the instrument or the computing device, etc. Updated images can be generated based on the adjusted exposure parameters, which can be further analyzed an adjusted. For example, in the case of the manual input, the technician can visually inspect and analyze the updated images in order to further adjust the exposure parameters. For example, in the case of the processor or computing device, the trained model can receive the updated images as an input and use the trained model to further adjust the exposure parameters.

In one embodiment, the first exposure control process 130 (and the second exposure control process 145 and any additional exposure control processes) can be a neural network configured to receive image data as an input. An output of the neural network can be a determination regarding whether the images corresponding to the image data are informative frames. That is, for example, the first exposure control process 130 and the second exposure control process 145 can receive two of the obtained images and determine whether the images include the object of interest and whether there is a significant change for the object of interest between the two images. For example, the first exposure control process 130 and the second exposure control process 145 can determine whether the difference between the first location of the object of interest in the first image 105 and the second location of the object of interest in the second image 110 is greater than the predetermined threshold.

In one embodiment, the image data input for the first exposure control process 130 and the second exposure control process 145 can include 2 or more images (or frames). The images or frames can also be used as an input into an image restoration process, the image restoration process using a neural network in order to restore images. The resulting restored images, and the quality of the restored images, can be used as feedback to the acquisition parameter adjustment process. In particular, the quality of the restored images can be used as feedback to the exposure control processes (described below).

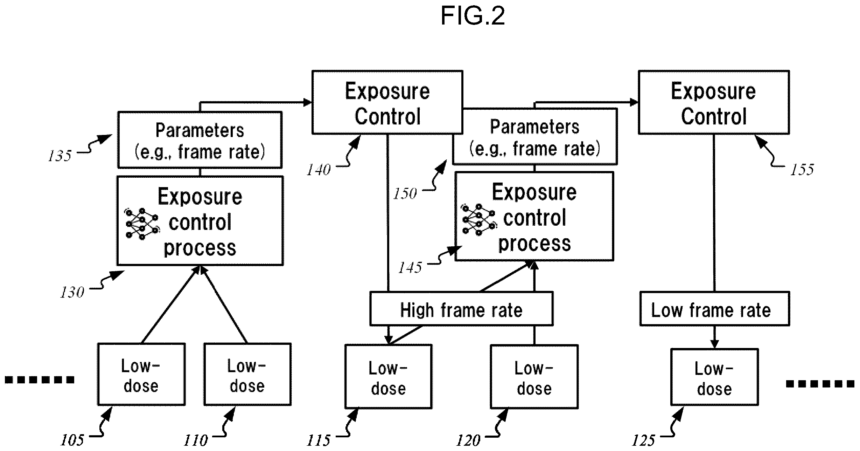

FIG. 2 is a schematic describing the acquisition parameter adjustment process via frame rate adjustment, according to an embodiment of the present disclosure. In one embodiment, the first image 105 can be obtained via the fluoroscopy system with the first exposure setting. Notably, the first image 105 can be a low-dose image, as shown. The low dose of the first image 105 can be understood to mean that the first image 105 was obtained using the exposure parameter that includes the low exposure or dose setting for the radiation source. The first image 105 can be analyzed by the processing circuitry to identify any object of interest disposed in the first image 105, for example a stent. In the first image 105, the processing circuitry can determine the first location of the object of interest.

In one embodiment, the second image 110 can be obtained via the fluoroscopy system with the first exposure setting. The second image 110 can also be a low-dose image, as shown. The second image 110 can be analyzed by the processing circuitry to identify the object of interest disposed in the second image 110 and the second location of the object of interest. For example, the object of interest can again move between acquisitions and the second location of the object of interest is not the same as the first location. The difference between the first location and the second location can be used to determine whether the first image 105 and the second image 110 are informative frames.

In one embodiment, the first exposure control process 130 performed by the processing circuitry (the fluoroscopy system) can analyze the first image 105 and the second image 110 to determine whether the first image 105 and the second image 110 are informative frames. Again, the object of interest (or multiple objects of interest) can be determined in the first image 105 and the second image 110, as well as the corresponding first location and the corresponding second location. Upon determining that the difference between the first location of the object of interest in the first image 105 and the second location of the object of interest in the second image 110 is greater than the predetermined threshold, the exposure control process can determine that the first image 105 and the second image 110 are informative frames. That is, the first image 105 and the second image 110 include a change in the object of interest that can be imaged at a higher dose or exposure in order to obtain an image with less noise compared to the same image obtained at a lower dose. As previously described, denoising need not be the target goal of increasing the exposure, and other target goals can be desired.

In one embodiment, upon determining that the first image 105 and the second image 110 are informative frames, the fluoroscopy system can adjust the first exposure parameters 135 via the first exposure control 140. The first exposure parameters 135 can be adjusted based on the informative frames, such as the exposure parameters of the informative frames, the object of interest in the informative frames, and determined image parameters of the informative frames (brightness, contrast, etc.). The first exposure control 140 can be configured to adjust, for example, the dose, voltage, frame rate, filter, etc. in the first exposure parameters 135. As shown in FIG. 2, the first exposure control 140 can increase the frame rate, followed by acquisition of the third image 115. Thus, the third image 115 can be labeled as still a low-dose image, but acquired at the high frame rate. Similarly, the fourth image 120 can be obtained, also at the high frame rate.

In one embodiment, upon determining that the first image 105 and the second image 110 are not informative frames, the fluoroscopy system can maintain the current first exposure parameters 135 used to obtain the subsequent images, which can continue to be considered low-dose images and also at the low frame rate. For example, the fluoroscopy system can determine that the difference between the first location of the object of interest in the first image 105 and the second location of the object of interest in the second image 110 is not greater than the predetermined threshold, and therefore maintain the low frame rate (and dose) used to obtain the images.

In one embodiment, the second exposure control process 145 performed by the processing circuitry can analyze the third image 115 and the fourth image 120 to determine whether the third image 115 and the fourth image 120 are informative frames. Again, the object of interest (or multiple objects of interest) can be determined in the third image 115 and the fourth image 120, and the corresponding third location for the object of interest in the third image 115 and the corresponding fourth location for the object of interest in the fourth image 120. Upon determining that the difference between the third location of the object of interest in the third image 115 and the fourth location of the object of interest in the fourth image 120 is not greater than the predetermined threshold, the exposure control process can determine that the third image 115 and the fourth image 120 are not informative frames. That is, it is possible to determine that the third image 115 and the fourth image 120 have negligible changes or no changes in the object of interest.

In one embodiment, upon determining that the third image 115 and the fourth image 120 are not informative frames, the fluoroscopy system can adjust second exposure parameters 150 via the second exposure control 155. The second exposure parameters 150 can be adjusted back to a low frame rate, or a frame rate less frequent than a frame rate used to obtain the third image 115 and the fourth image 120 that were obtained using the high frame rate. As shown in FIG. 2, the second exposure control 155 can decrease the frame rate, followed by acquisition of the fifth image 125.

In one embodiment, although described as being parameters adjusted separately, the aforementioned parameters can be adjusted together. That is, the dose level and the frame rate can be adjusted together in order to obtain subsequent images that includes less noise. By extension, the dose can be adjusted in combination with a different parameters, such as the acquisition time.

In FIG. 1 and FIG. 2, an example in which determining whether a frame is informative is performed based on a plurality of images is described, however, determining whether a frame is informative may be performed based on a single image. For example, whether a frame is informative may be determined based on the first image 105 illustrated in FIG. 1 or FIG. 2, and based on the result of the determination, adjusted exposure parameters may be acquired. As an example, it is possible to determine that the first image 105 is an informative frame if an object of interest such as a stent or a contrast agent is included in the first image 105. If the first image 105 is an informative frame, the exposure parameters can be adjusted to acquire the second image 110 with a higher dose and frame rate.

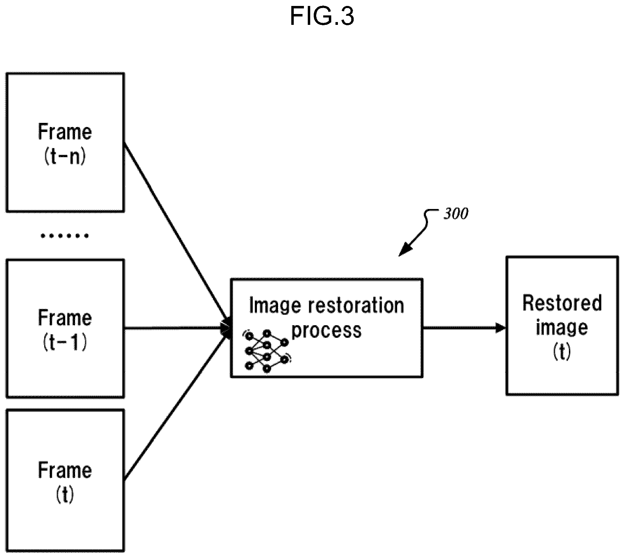

FIG. 3 is a schematic of an image restoration process 300, according to an embodiment of the present disclosure. As previously mentioned, a sequence of images or frames can be used as the input into the image restoration process 300. The image restoration process 300 can include a neural network trained to restore the images. In an embodiment, the neural network for restoring the images can be used to improve the image quality. The image restoration process 300 can include denoising, deblurring, artifact removal, etc. The image data input for the image restoration process 300 can include 2 or more images (or frames). As shown, a current frame (t), a previous frame (t-1), and up to any number of frames (t-n) can be used as the input into the image restoration process 300, which generates a restored image. Again, the resulting restored images, and the quality of the restored images, can be used as feedback to the acquisition parameter adjustment process. In particular, the quality of the restored images can be used as feedback to the exposure control processes.

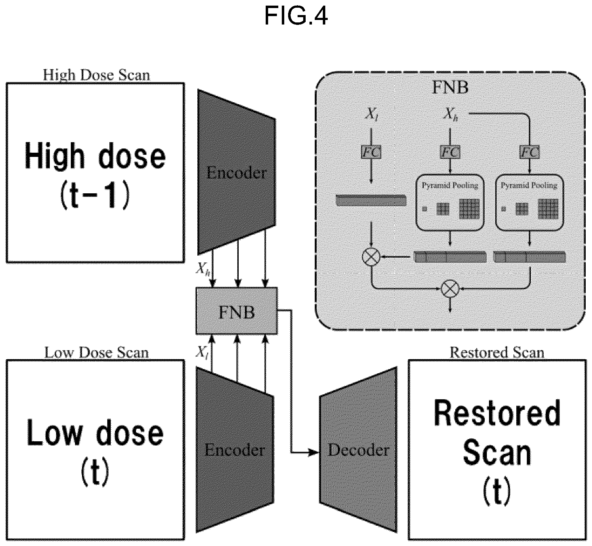

FIG. 4 is a schematic describing a neural network architecture for image restoration, according to an embodiment of the present disclosure. In one embodiment, the neural network input can include the image data, which can include a sequence of images, such as those obtained by the fluoroscopy system. The neural network architecture can include a first encoder and a second encoder configured to extract features from the images with different exposure parameters. The encoders generally can be configured to transform high-dimensional input data into a lower-dimensional representation that retains essential information for further processing in various machine learning tasks. For example, the first encoder can be configured to extract features from the high-dose images, and the second encoder can be configured to extract features from the low-dose images. The encoders can be used in various other neural network architectures, including autoencoders, convolutional neural networks (CNNs), and recurrent neural networks (RNNs).

In one embodiment, the neural network architecture can also include a Non-local Block, or in particular, a Fuse Non-local Block (FNB). A Non-Local Block can be an image block module used in neural networks that wraps a non-local operation or captures long-range dependencies in data, such as image data. The FNB can function by allowing each element in the input to interact with all other elements regardless of the spatial or temporal distance between the elements, while also fusing the features from the images with different exposure settings. For example, the FNB can help the neural network determine any connections between the images by calculating a similarity value between all pairs of positions in the images. The similarity value can be used to adjust an importance or score of each pixel in the image, so that pixels that are more related to one another are given more weight.

In one embodiment, the neural network can include a decoder configured to accept the fused feature from the images as an input in order to generate and output the restored image. For example, the neural network architecture can use an autoencoder including an encoder and a decoder, where the encoder maps input data to a lower-dimensional latent space representation (latent vectors), and the decoder attempts to reconstruct the original data from the lower-dimensional latent space representation. By attempting to match its outputs to its inputs, the autoencoder can learn to grasp relevant information and ignore insignificant data. Autoencoders can be used in tasks such as data compression, denoising, and anomaly detection.

Notably, the neural network for the first exposure control process 130 (or the second exposure control process 145) and the neural network for the image restoration process 300 can be trained alternatively.

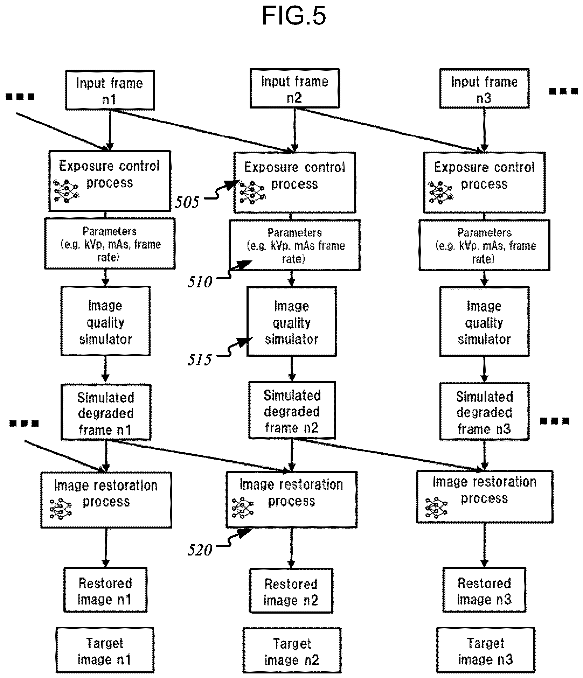

To this end, FIG. 5 is a schematic describing the neural network training, according to an embodiment of the present disclosure. In one embodiment, the neural network for the image restoration process 300 can be fixed while the neural network for the first exposure control process 130 can be trained. With reference to FIG. 5, a first input frame n1 and a second input frame n2 can be inputs for an exposure control process 505 having a neural network for training. An image quality simulator 515 can be configured to simulate a simulated degraded frame n2. The image quality simulator 515 can simulate the images based on the input including exposure parameters 510 (similar to the exposure parameters described previously). A loss function for the exposure control process 505 can be defined to (i) maximize an image quality of the entire sequence of the images, and (ii) minimize the dose level used to obtain the entire sequence of the images. The loss function for the exposure control process 505 can be described by, for example, the following equation (1)

$$L = \frac{1}{n}\sum_{i=0\ to\ n} |pred_i - target_i| + \frac{1}{n}\sum_{i=0\ to\ n} dose_i \qquad \text{...(1)}$$

where n is the total frame number of the sequence. As shown, multiple of the sequences of the images can be used for the neural network training.

In one embodiment, the neural network for the exposure control process 505 can be fixed while the neural network for an image restoration process 520 can be trained. As shown in FIG. 5, a simulated degraded frame n1 and the simulated degraded frame n2 can be inputs for the image restoration process 520 having a neural network for training. The output of the image restoration process 520 can be a restored image n2. This can be compared to a target image n2, and further training and modifications to the neural networks can be performed to improve the accuracy of the output of the image restoration process 520. A loss function for the image restoration process 520 can be defined to maximize the image quality of each individual image or frame. The loss function for the image restoration process 520 process can be described by, for example, the following equation (2)

$$L = \sum |pred_i - target_i| \qquad \text{...(2)}$$

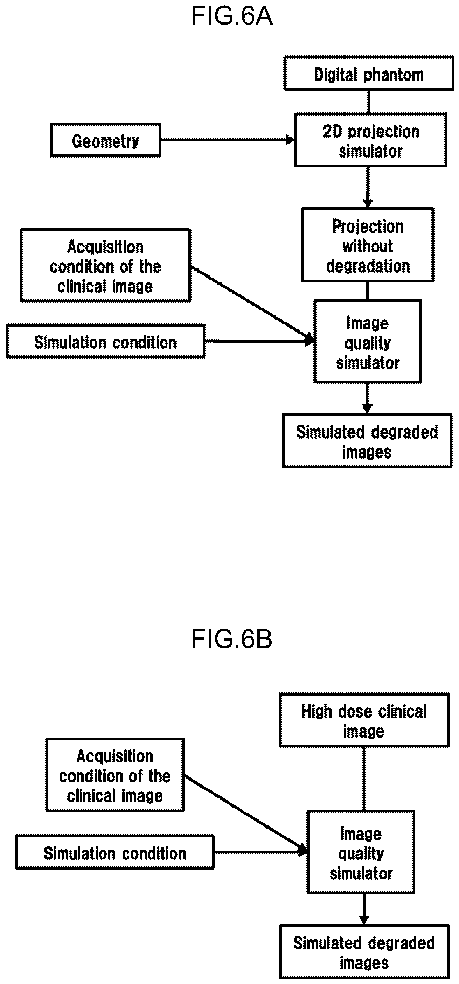

The training datasets can be generated according to various processes. To this end, FIG. 6A and FIG. 6B are schematics describing training dataset generation, according to an embodiment of the present disclosure.

In one embodiment, FIG. 6A describes the use of phantoms (digital or real). The phantom can be an object with known parameters (such as size, geometry, density, etc.) that is imaged using the fluoroscopy system to evaluate, analyze, and optimize the performance of the fluoroscopy system. Notably, the phantom can be used with 3D device models to simulate interventional procedures, and noise can be added to high dose and low dose frames. As shown, data from the phantom, along with the known characteristics of the phantom (e.g., the phantom geometry) can be used as inputs for a 2D projection simulator to generate projection data (an image) without degradation included. Known acquisition parameters and simulation parameters, along with the projection data without degradation can be used as inputs for an image quality simulator to generate simulated degraded images for the training datasets.

In one embodiment, FIG. 6B shows the use of high-dose frames with known additions of noise. A high-dose image, such as a high-dose image obtained in a clinical setting, along with the acquisition parameters and the simulation parameters as the inputs for the image quality simulator to generate the simulated degraded images for the training datasets.

In an embodiment, the neural network for the exposure control process 505 can be trained together with the neural network for the image restoration process 520 using a training sequence of images in a training process. During the training process, inputs to the neural network for the image restoration process 520 can be based on outputs of the neural network for the exposure control process 505, and the neural network for the image restoration process 520 outputs a sequence of restored images based on the training sequence of images. The neural network for the exposure control process 505 can be trained using a first loss function having a first term representing a value relating to total dosage used in acquiring the training sequence of images and a second term representing a value relating to total error in the sequence of restored images, as described above. The neural network for the image restoration process 520 can be trained using a second loss function having a term representing the value relating to total error in the sequence of restored images, as described above. Notably, the neural network for the exposure control process 505 can be trained alternatively with the neural network for the image restoration process 520.

As shown in FIG. 5, in the training process, the neural network in the exposure control process 505 receives the input frame n1 and the input frame n2 and outputs the exposure parameters 510. The neural network of the exposure control process 505 is an example of the first model. The input frame n1 and the input frame n2 are each an example of the first training image. The exposure parameters 510 are an example of a set of training exposure parameters.

In the training process, the neural network in the image restoration process 520 receives a pseudo-degraded frame n1 and a pseudo degraded frame n2 to output a restored image n2. The neural network of the image restoration process 520 is an example of the second model. The pseudo degraded frame n1 and the pseudo-degraded frame n2 are each an example of second training images acquired based on a set of training exposure parameters. The restored image n2 is an example of a training restored image.

In the training process, the first model is updated based on a comprehensive evaluation of the X-ray radiation dose corresponding to the set of training exposure parameters and the image quality of a training restored image. For example, the first model is trained to minimize the loss function expressed in equation (1).

Specifically, when the X-ray radiation dose corresponding to the set of training exposure parameters is large, the value of the second term on the right-hand side of equation (1) becomes larger. In contrast, if the X-ray radiation dose corresponding to the training exposure parameter set is small, the image quality of the second training image acquired based on the set of training exposure parameters is degraded. Generally, the image quality of the input image is degraded, so that the accuracy of the image restoration process is degraded. That is, the image quality of the training restored image is also degraded. As a result, the error, for example, between a target image n2 and the restored image n2 shown in FIG. 5 becomes larger, and the value of the first term on the right-hand side of equation (1) becomes larger. Thus, by minimizing the loss function expressed in equation (1), the first model can be trained so that the two objectives of a reduction in radiation exposure and an improvement in image quality are accomplished simultaneously.

By alternately training the first and second models in the training process, the two objectives of a reduction in radiation exposure and an improvement in image quality can be accomplished at a higher level. That is, by training the first model, a reduction in radiation exposure and an improvement in image quality are tentatively accomplished. Second, by training the second model, the performance of image restoration is improved to a satisfactory level, even for an image acquired under a lower dose condition. By further training the first model here, the radiation exposure is further reduced while image quality is maintained by the second model with improved performance.

The first model may be further trained to improve image quality for informative frames. That is, the first model may be updated based on a comprehensive evaluation of the informativeness of the first training image, the X-ray radiation dose corresponding to the set of training exposure parameters, and the image quality of the training restored image.

For example, in FIG. 5, by identifying the position of the object of interest in each of the input frame n1 and the input frame n2 and calculating the amount of movement of the object of interest, an indicator representing the informativeness of the input frame n1 and the input frame n2 can be calculated. Then, for example, the first model is trained by minimizing the loss function after transformation that multiplies the first term on the right-hand side of equation (1) by the calculated index. This allows the first model to be trained to give higher priority to improving image quality when the input frame n1 and the input frame n2 are informative frames. In contrast, if the input frame n1 and the input frame n2 are not informative frames, the first model can be trained to give higher priority to reducing the X-ray radiation dose.

In FIG. 5, an example where the input frame n1 and the input frame n2 are input into the first model has been described. That is, an example of multiple training images being input into the first model has been described. However, embodiments are not limited thereto, and there may be only one training image input into the first model. For example, in FIG. 5, the neural network in the exposure control process 505 may only receive the input frame n2 and output the exposure parameters 510.

In FIG. 5, an example of the pseudo-degraded frame n1 and the pseudo-degraded frame n2 being input into the second model has been described. That is, an example of multiple training images being input into the second model has been described. However, embodiments are not limited thereto, and there may be only one training image input into the second model. For example, in FIG. 5, the neural network of the image restoration process 520 may only receive the input of pseudo-degraded frame n2 and output the restored image n2.

In FIG. 5, an example of a pseudo-degraded frame being input into the second model has been described. That is, as shown in FIG. 6A and FIG. 6B, for example, an example in which a low-quality image is simulated based on a high-quality image and the simulated pseudo-degraded frame is input into the second model has been described. However, embodiments are not limited thereto.

That is, the input for the second model may be acquired images not relying on simulation. For example, instead of the pseudo-degraded frame shown in FIG. 5, a clinical image or a phantom image acquired under a low-dose condition may be used as input to the neural network of the image restoration process 520. In this case, an image acquired from the same subject or phantom under a high dose condition can be used as the target image n2.

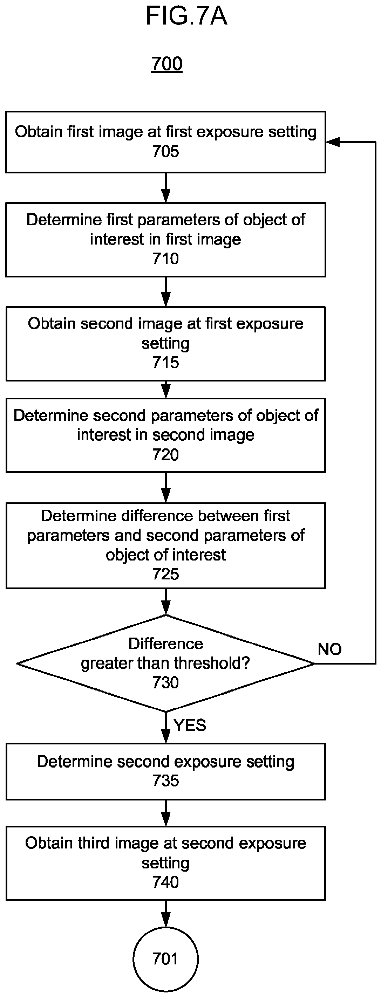

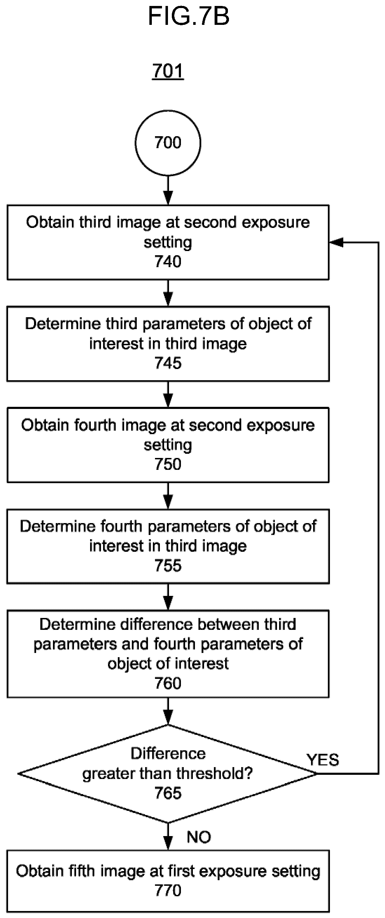

FIG. 7A and FIG. 7B show a non-limiting example of a flow chart for a method 700 of acquisition parameter adjustment, according to an embodiment of the present disclosure.

In an embodiment, at step 705, the fluoroscopy system can obtain a first image including projection data representing an intensity of X-rays, emitted from an X-ray source, detected by a plurality of detectors at a first X-ray exposure setting. For example, the first X-ray exposure setting is at a low dose or low radiation level, and the first image is a low-dose image.

In an embodiment, at step 710, the fluoroscopy system can determine whether an object of interest is disposed in the first image and, upon determining the first image includes the object of interest, determine first parameters of the first object of interest in the first image. For example, the first parameters of the first object of interest includes a first location of the first object of interest.

In an embodiment, at step 715, the fluoroscopy system can obtain a second image including the projection data representing the intensity of the X-rays, emitted from the X-ray source, detected by the plurality of detectors at the first X-ray exposure setting. Thus, the second image can also be a low-dose image.

In an embodiment, at step 720, the fluoroscopy system can determine second parameters of the first object of interest in the second image. For example, the second parameters of the first object of interest includes a second location of the first object of interest.

In an embodiment, at step 725, the fluoroscopy system can determine a difference between the first parameters and the second parameters of the first object of interest. For example, the difference between the first parameters and the second parameters is the difference (a distance) between the first location and the second location of the first object of interest.

In an embodiment, at step 730, the fluoroscopy system can determine whether the difference between the first parameters and the second parameters is greater than a predetermined threshold. That is, the fluoroscopy system can determine whether the first image and the second image are informative frames, or images with significant events occurring. For example, the predetermined threshold is determined based on a distance between the first location and the second location of the first object of interest. For example, the predetermined threshold is determined based on a size difference between a first size of the first object of interest in the first image and a second size of the first object of interest in the second image. For example, the predetermined threshold is determined based on a density difference between a first density of the first object of interest in the first image and a second density of the first object of interest in the second image.

In an embodiment, at step 730, upon determining the difference is not greater than the predetermined threshold, the fluoroscopy system can continue to obtain images at the first exposure setting by proceeding to step 705. That is, the first image and the second image are not informative frames, and therefore the low dose imaging can be used to continue imaging the first object of interest.

In an embodiment, at step 735, upon determining the difference is greater than the predetermined threshold, the fluoroscopy system can determine a second X-ray exposure setting for use in obtaining a third image. That is, the first image and the second image are informative frames with significant events occurring in the images. For example, the object of interest has had a significant repositioning.

In an embodiment, at step 740, the fluoroscopy system can obtain the third image including the projection data representing the intensity of the X-rays, emitted from the X-ray source, detected by the plurality of detectors at the second X-ray exposure setting. For example, the second X-ray exposure setting is at a high dose or high radiation level for obtaining the third image with less noise or higher image quality, and the third image can be a high-dose image.

With reference to FIG. 7B, in an embodiment, at step 745, the fluoroscopy system can determine third parameters of the first object of interest in the third image. For example, the third parameters of the first object of interest includes a third location of the first object of interest.

In an embodiment, at step 750, the fluoroscopy system can obtain a fourth image including the projection data representing the intensity of the X-rays, emitted from the X-ray source, detected by the plurality of detectors at the second X-ray exposure setting. Thus, the fourth image can also be a high-dose image.

In an embodiment, at step 755, the fluoroscopy system can determine fourth parameters of the first object of interest in the fourth image. For example, the fourth parameters of the first object of interest includes a fourth location of the first object of interest.

In an embodiment, at step 760, the fluoroscopy system can determine a difference between the third parameters and the fourth parameters of the first object of interest. For example, the difference between the third parameters and the fourth parameters is the difference (a distance) between the third location and the fourth location of the first object of interest.

In an embodiment, at step 765, the fluoroscopy system can determine whether the difference between the third parameters and the fourth parameters is greater than the predetermined threshold. That is, the fluoroscopy system can determine whether the third image and the fourth image are informative frames, or images with significant events occurring.

In an embodiment, at step 765, upon determining the difference is greater than the predetermined threshold, the fluoroscopy system can continue to obtain images at the second exposure setting by proceeding to step 740. That is, the third image and the fourth image are still informative frames, and therefore the high dose imaging can be used to continue imaging the first object of interest.

In an embodiment, at step 765, upon determining the difference is not greater than the predetermined threshold, the fluoroscopy system can revert to obtaining images, such as a fifth image, at the first exposure setting by proceeding to step 770. That is, the third image and the fourth image are not informative frames, and therefore the low dose imaging can be used to image the first object of interest again. It may be appreciated that at step 770, a third X-ray exposure setting can be determined for use in obtaining the fifth image instead of reverting to the first X-ray exposure setting.

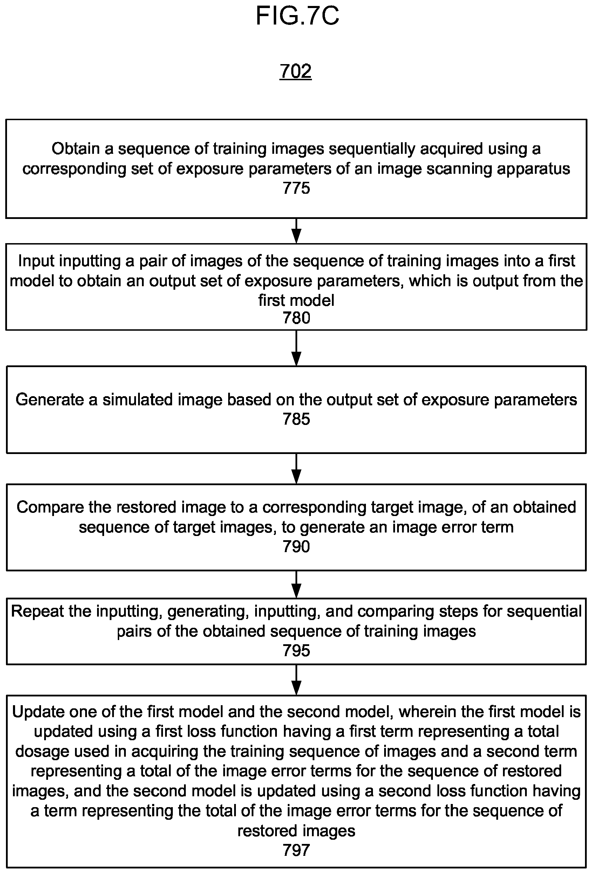

FIG. 7C shows a non-limiting example of a flow chart for a method 702 of acquisition parameter adjustment, according to an embodiment of the present disclosure.

In an embodiment, at step 775, a sequence of training images is obtained, the sequence of training images sequentially acquired using a corresponding set of exposure parameters of an image scanning apparatus.

In an embodiment, at step 780, a pair of images of the sequence of training images is input into a first model to obtain an output set of exposure parameters, which is output from the first model.

In an embodiment, at step 785, a simulated image is generated based on the output set of exposure parameters. Further, the generated simulation image is input to the second model to obtain the restored image output from the second model.

In an embodiment, at step 790, the restored image is compared to a corresponding target image, of a acquired sequence of target images, to generate an image error term.

In an embodiment, at step 795, the inputting, generating, inputting, and comparing steps are repeated for sequential pairs of the acquired sequence of training images.

In an embodiment, at step 797, one of the first model and the second model is updated, the first model is updated using a second loss function having a first term representing a total dosage used in acquiring the training sequence of images and a second term representing a total of the image error terms for the sequence of restored images, and updating the second model using a second loss function having a term representing the total of the image error terms for the sequence of restored images.

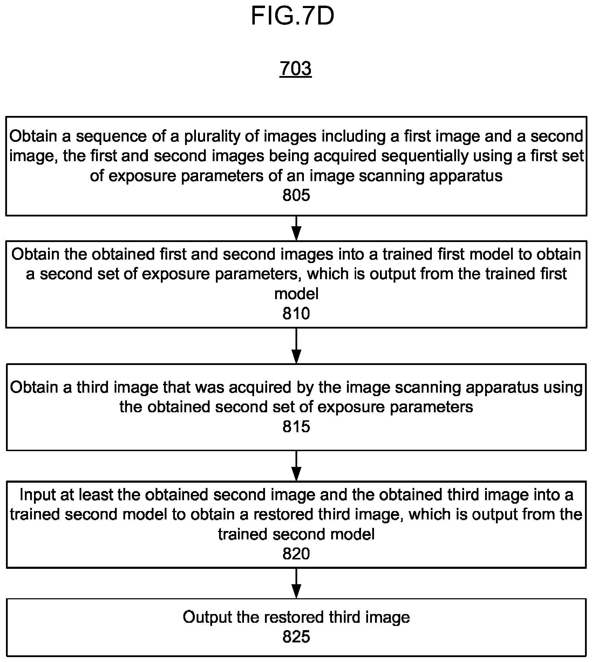

FIG. 7D shows a non-limiting example of a flow chart for a method including acquisition parameter adjustment and image restoration using a trained model, according to an embodiment of the present disclosure.

In an embodiment, at step 805, a sequence of a plurality of images including a first image and a second image is obtained. The first and second images are acquired sequentially using a first set of exposure parameters of an image scanning apparatus.

In an embodiment, at step 810, the obtained first and second images are input into a trained first model to obtain a second set of exposure parameters, which is output from the trained first model. That is, at step 810, multiple X-ray images acquired using a first set of exposure parameters are input to the first model to obtain a second set of exposure parameters.

The multiple X-ray images input to the first model at step 810 may be multiple adjacent frames of X-ray images. For example, the second image input to the first model may be the image of the next frame of the first image. In this case, the informativeness can be evaluated by calculating the amount of movement of the object of interest, etc., between the adjacent frames, and the evaluation results can be input to the first model. Conversely, there may be only one X-ray image input to the first model.

In an embodiment, at step 815, a third image is obtained that was acquired by the image scanning apparatus using the obtained second set of exposure parameters.

In an embodiment, at step 820, at least the obtained second image and the obtained third image are input into a trained second model to obtain a restored third image, which is output from the trained second model. There may be only one X-ray image input to the second model. For example, at step 820 in FIG. 7D, the third image is input into the trained second model to obtain the restored third image, which is output from the trained second model.

In an embodiment, at step 825, the restored third image is output.

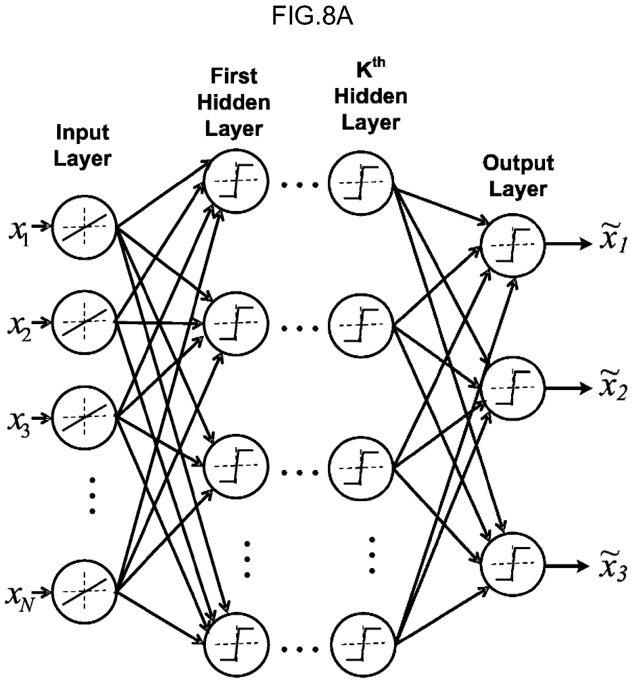

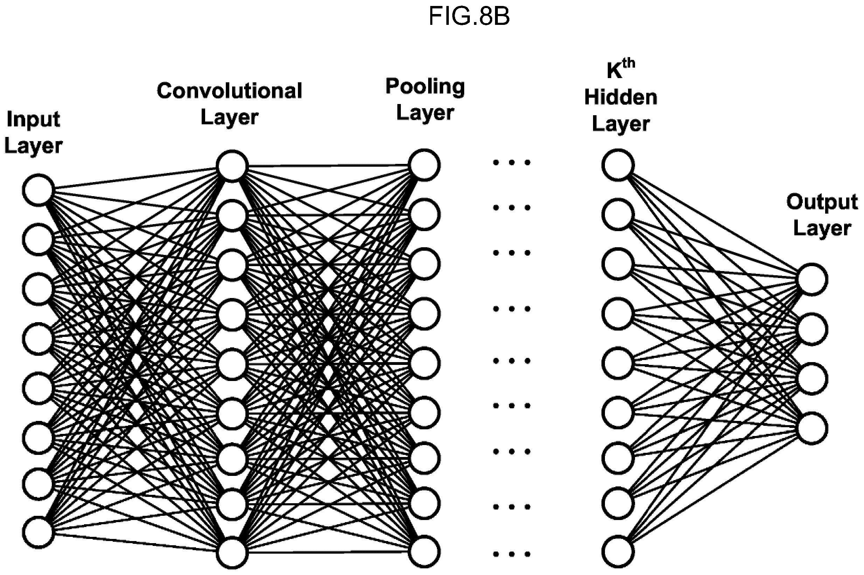

FIG. 8A and 8B show various examples of a deep learning (DL) network.

FIG. 8A shows an example of a general artificial neural network (ANN) having N inputs, K hidden layers, and three outputs. Each layer is made up of nodes (also called neurons), and each node performs a weighted sum of the inputs and compares the result of the weighted sum to a threshold to generate an output. ANNs make up a class of functions for which the members of the class are obtained by varying thresholds, connection weights, or specifics of the architecture such as the number of nodes and/or their connectivity. The nodes in an ANN can be referred to as neurons (or as neuronal nodes), and the neurons can have inter-connections between the different layers of the ANN system. The simplest ANN has three layers, and is called an autoencoder. The DL network generally has more than three layers of neurons, and has as many outputs neurons $\bar{x}_N$ as input neurons, wherein $N$ is the number of pixels in the reconstructed image (sinogram). The synapses (i.e., the connections between neurons) store values called "weights" (also interchangeably referred to as "coefficients" or "weighting coefficients") that manipulate the data in the calculations. The outputs of the ANN depend on three types of parameters: (i) the interconnection pattern between the different layers of neurons, (ii) the learning process for updating the weights of the interconnections, and (iii) the activation function that converts a neuron's weighted input to its output activation.

Mathematically, a neuron's network function $m(x)$ is defined as a composition of other functions $n_i(x)$, which can further be defined as a composition of other functions. This can be conveniently represented as a network structure, with arrows depicting the dependencies between variables, as shown in the figures. For example, the ANN can use a nonlinear weighted sum, wherein m(x) is described by the following equation (3),

$$m(x) = K(\Sigma_i w_i n_i(x)) \qquad ...(3)$$

where K (commonly referred to as the activation function) is some predefined function, such as the hyperbolic tangent.

In FIG. 8A (and similarly in FIG. 8B), the neurons (i.e., nodes) are depicted by circles around a threshold function. In certain implementations, the DL network is a feedforward network as described in FIG. 8A and 8B (e.g., it can be represented as a directed acyclic graph).

The DL network operates to achieve a specific task, such as denoising an image, by searching within the class of functions $F$ to learn, using a set of observations, to find $m* \in F$ which solves the specific task in some optimal sense. For example, in certain implementations, this can be achieved by defining a cost function $C: F \rightarrow m$ such that, for the optimal solution $m*$, $C(m*) \leq C(m) \forall m \in F$ (i.e., no solution has a cost less than the cost of the optimal solution). The cost function $C$ is a measure of how far away a particular solution is from an optimal solution to the problem to be solved (e.g., the error). Learning algorithms iteratively search through the solution space to find a function that has the smallest possible cost. In certain implementations, the cost is minimized over a sample of the data (i.e., the training data).

FIG. 8B shows a non-limiting example in which the DL network is a convolutional neural network (CNN). CNNs are type of ANN that has beneficial properties for image processing, and, therefore, have specially relevancy for the applications of image denoising and sinogram restoration. CNNs use feed-forward ANNs in which the connectivity pattern between neurons can represent convolutions in image processing. For example, CNNs can be used for image-processing optimization by using multiple layers of small neuron collections which process portions of the input image, called receptive fields. The outputs of these collections can then tiled so that they overlap, to obtain a better representation of the original image. This processing pattern can be repeated over multiple layers having alternating convolution and pooling layers.

As generally applied above, following after a convolution layer, a CNN can include local and/or global pooling layers which combine the outputs of neuron clusters in the convolution layers. Additionally, in certain implementations, the CNN can also include various combinations of convolutional and fully connected layers, with pointwise nonlinearity applied at the end of or after each layer.

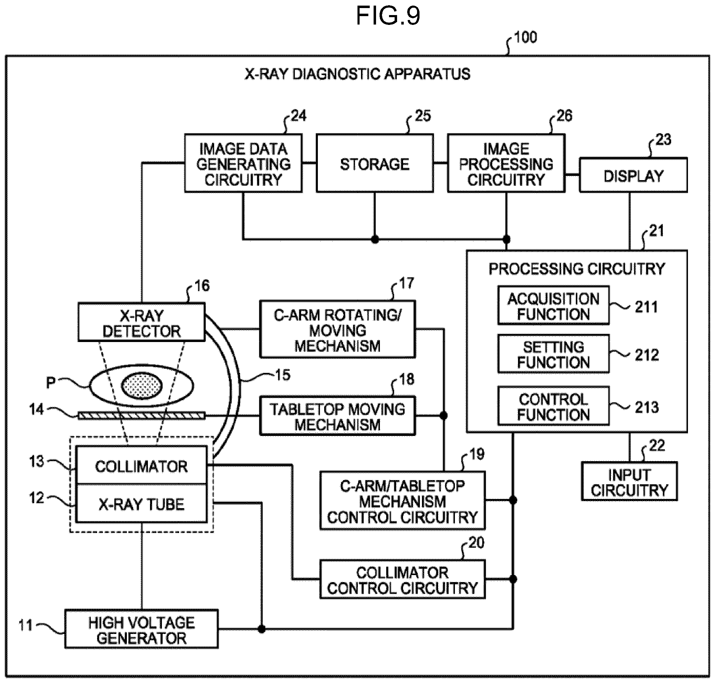

FIG. 9 is a diagram illustrating a configuration of an X-ray diagnostic apparatus 100 according to an embodiment. As illustrated, the X-ray diagnostic apparatus 100 includes a high voltage generator 11, an X-ray tube 12, a collimator 13, a tabletop 14, a C-arm 15, an X-ray detector 16, a C-arm rotating/moving mechanism 17, a tabletop moving mechanism 18, C-arm/tabletop mechanism control circuitry 19, collimator control circuitry 20, processing circuitry 21, input circuitry 22, a display 23, image data generating circuitry 24, a storage 25, and image processing circuitry 26.

In the X-ray diagnostic apparatus 100, each processing function is stored in the storage 25 in the form of a computer program executable by a computer. The C-arm/tabletop mechanism control circuitry 19, the collimator control circuitry 20, the processing circuitry 21, the image data generating circuitry 24, and the image processing circuitry 26 are a processor that reads a computer program from the storage 25 and executes the computer program to implement the function corresponding to the computer program. In other words, each circuit in a state in which a computer program is read has the function corresponding to the read computer program.

The term "processor" used in the description above means, for example, a central processing unit (CPU), a graphics processing unit (GPU), or a circuit such as an application specific integrated circuit (ASIC) and a programmable logic device (for example, simple programmable logic device (SPLD), complex programmable logic device (CPLD), and a field programmable gate array (FPGA)). The processor reads and executes a computer program stored in the storage circuit to implement the function. The computer program may be directly built in a circuit in the processor, rather than being stored in a storage circuit. In this case, the processor implements the function by reading and executing the computer program built in the circuit. Each processor in the present embodiment may not be configured as a single circuit, but a plurality of independent circuits may be combined into a single processor, which implements the function.

The high voltage generator 11 generates high voltage and supplies the generated high voltage to the X-ray tube 12, under control by the processing circuitry 21. The X-ray tube 12 generates X-rays using the high voltage supplied from the high voltage generator 11.

The collimator 13 narrows X-rays produced by the X-ray tube 12 such that the X-rays are selectively applied to a region of interest of a subject P, under control by the collimator control circuitry 20. For example, the collimator 13 has four slidable collimator blades. The collimator 13 allows these collimator blades to slide under control by the collimator control circuitry 20 and thereby narrows the X-rays produced by the X-ray tube 12 to apply the narrowed X-rays to the subject P. The collimator 13 also includes an additional filter for adjusting the radiation quality. The additional filter is set, for example, depending on tests. The tabletop 14 is a bed on which the subject P lies and is disposed on a not-illustrated table (couch). The subject P is not included in the X-ray diagnostic apparatus 100.

The X-ray detector 16 detects X-rays transmitted through the subject P. For example, the X-ray detector 16 includes detecting elements arranged in a matrix. Each detecting element converts X-rays transmitted through the subject P into an electrical signal, accumulates the electrical signals, and transmits the accumulated electrical signals to the image data generating circuitry 24. In the X-ray diagnostic apparatus 100, the X-ray tube 12, the collimator 13, the X-ray detector 16, and other imaging mechanisms for capturing X-ray images, are also referred to as image scanning device or X-ray device.

The C-arm 15 holds the X-ray tube 12, the collimator 13, and the X-ray detector 16. The C-arm 15 is rotated fast like a propeller around the subject P lying on the tabletop 14, by a motor provided at a support (not illustrated). Here, the C-arm 15 is rotatably supported with respect to three axes orthogonal to each other, namely, the XYZ axes, and is rotated individually in each axis by a not-illustrated driver. The X-ray tube 12 and the collimator 13 are disposed to be opposed to the X-ray detector 16 by means of the C-arm 15 with the subject P interposed. Although the X-ray diagnostic apparatus 100 is a single-plane system by way of example, embodiments are not limited thereto and may employ a biplane system. FIG. 9 illustrates an X-ray diagnostic apparatus 100 equipped with the C-arm 15, however, embodiments are not limited thereto and can be applied in the same manner to any type of X-ray diagnostic apparatus, for example, an X-ray TX apparatus or the like.

The C-arm rotating/moving mechanism 17 is a mechanism for rotating and moving the C-arm 15. The C-arm rotating/moving mechanism 17 can also change a source image receptor distance (SID) which is the distance between the X-ray tube 12 and the X-ray detector 16. The C-arm rotating/moving mechanism 17 can also rotate the X-ray detector 16 held by the C-arm 15. The tabletop moving mechanism 18 is a mechanism for moving the tabletop 14.

The C-arm/tabletop mechanism control circuitry 19 controls the C-arm rotating/moving mechanism 17 and the tabletop moving mechanism 18 under control by the processing circuitry 21 to adjust the rotation and movement of the C-arm 15 and the movement of the tabletop 14. For example, the C-arm/tabletop mechanism control circuitry 19 controls rotation imaging to collect projection data at a predetermined frame rate while rotating the C-arm 15, under control by the processing circuitry 21. The collimator control circuitry 20 controls the radiation range of X-rays applied to the subject P by adjusting the aperture of the collimator blades of the collimator 13, under control by the processing circuitry 21.

The image data generating circuitry 24 generates projection data using the electrical signal obtained through conversion of X-rays by the X-ray detector 16 and stores the generated projection data into the storage 25. For example, the image data generating circuitry 24 performs current-voltage conversion, analog-digital (A/D) conversion, and parallel-serial conversion on the electrical signal received from the X-ray detector 16 to generate projection data. The image data generating circuitry 24 then stores the generated projection data into the storage 25.

The storage 25 accepts and stores the projection data generated by the image data generating circuitry 24. The storage 25 stores computer programs corresponding to various functions to be read and executed by the circuits illustrated. As an example, the storage 25 stores a computer program corresponding to an acquisition function 211, a computer program corresponding to a setting function 212, and a computer program corresponding to a control function 213 to be read and executed by the processing circuitry 21.

The image processing circuitry 26 performs various image processing on the projection data stored in the storage 25 to generate an X-ray image, under control by the processing circuitry 21 described later. Alternatively, the image processing circuitry 26 directly acquires projection data from the image data generating circuitry 24 and performs various image processing on the acquired projection data to generate an X-ray image, under control by the processing circuitry 21 described later. The image processing circuitry 26 may store the processed X-ray image into the storage 25. For example, the image processing circuitry 26 can execute various processing with image processing filters such as moving average (smoothing) filter, Gaussian filter, median filter, recursive filter, and bandpass filter.

The image processing circuitry 26 also forms reconstruction data (volume data) from projection data collected by rotation imaging. The image processing circuitry 26 then stores the reconstructed volume data into the storage 25. The image processing circuitry 26 generates a three-dimensional image from volume data. For example, the image processing circuitry 26 generates a volume rendering image or a multi planar reconstruction (MPR) image from volume data. The image processing circuitry 26 then stores the generated three-dimensional image into the storage 25.

The input circuitry 22 is implemented by, for example, a trackball, a switch button, a mouse, and a keyboard for setting a predetermined region (for example, a region of interest such as a section concerned), and a footswitch for emitting X-rays. The input circuitry 22 is connected to the processing circuitry 21 and converts an input operation accepted from the operator into an electrical signal for output to the processing circuitry 21. The display 23 displays a graphical user interface (GUI) for accepting the operator's instruction and a variety of images generated by the image processing circuitry 26.

The processing circuitry 21 controls the operation of the entire X-ray diagnostic apparatus 100. Specifically, the processing circuitry 21 executes various processing by reading a computer program corresponding to the control function 213 for controlling the entire apparatus from the storage 25 for execution. For example, the control function 213 controls an X-ray radiation dose to be applied to the subject P and ON/OFF by controlling the high voltage generator 11 in accordance with the operator's instruction forwarded from the input circuitry 22 and adjusting the voltage supplied to the X-ray tube 12. For example, the control function 213 controls the C-arm/tabletop mechanism control circuitry 19 in accordance with the operator's instruction and adjusts the rotation and movement of the C-arm 15 and the movement of the tabletop 14. For example, the control function 213 controls the radiation range of X-rays applied to the subject P by controlling the collimator control circuitry 20 in accordance with the operator's instruction and adjusting the aperture of the collimator blades of the collimator 13.

The control function 213 also controls, for example, the image data generation processing by the image data generating circuitry 24 and the image processing or the analysis processing by the image processing circuitry 26 in accordance with the operator's instruction. The control function 213 also performs control such that a GUI for accepting the operator's instruction or an image stored in the storage 25 appears on the display 23.

In an embodiment, the processing circuitry 21 executes the control function 213 described above as well as the acquisition function 211 and the setting function 212. It is noted that the processing circuitry 21 is an example of the processing circuitry in the claims. For example, the acquisition function 211 is an example of an acquisition unit and inputs X-ray images acquired using the first set of exposure parameters into the first model to obtain the second set of exposure parameters. The setting function 212 is an example of a setting unit and inputs X-ray images acquired using the second set of exposure parameters into the second model trained together with the first model to obtain a restored image. The control function 213 is an example of a setting unit and outputs the restored image. The control function 213 may perform the various training processes described above. Alternatively, the various training processes described above may be performed by an external device different from the X-ray diagnostic apparatus 100, and the control function 213 may be configured to obtain a model trained by the external device.

Embodiments of the radiation detector apparatus data and the functional operations described in this specification can be implemented by digital electronic circuitry, in tangibly embodied computer software or firmware, in computer hardware, including the structures disclosed in this specification and their structural equivalents, or in combinations of one or more of them. Embodiments of the subject matter described in this specification can be implemented as one or more computer programs, i.e., one or more modules of computer program instructions encoded on a tangible non-transitory program carrier for execution by, or to control the operation of data processing apparatus, such as a networked device or server, user devices, and the like. The computer storage medium can be a machine-readable storage device, a machine-readable storage substrate, a random or serial access memory device, or a combination of one or more of them.

The term "data processing apparatus" refers to data processing hardware and may encompass all kinds of apparatus, devices, and machines for processing data, including by way of example a programmable processor, a computer, or multiple processors or computers. The apparatus can also be or further include special purpose logic circuitry, e.g., an FPGA (field programmable gate array) or an ASIC (application-specific integrated circuit). The apparatus can optionally include, in addition to hardware, code that creates an execution environment for computer programs, e.g., code that constitutes processor firmware, a protocol stack, a database management system, an operating system, or a combination of one or more of them.

A computer program, which may also be referred to or described as a program, software, a software application, a module, a software module, a script, or code, can be written in any form of programming language, including compiled or interpreted languages, or declarative or procedural languages, and it can be deployed in any form, including as a stand-alone program or as a module, component, Subroutine, or other unit suitable for use in a computing environment. A computer program may, but need not, correspond to a file in a file system. A program can be stored in a portion of a file that holds other programs or data, e.g., one or more scripts stored in a markup language document, in a single file dedicated to the program in question, or in multiple coordinated files, e.g., files that store one or more modules, subprograms, or portions of code. A computer program can be deployed to be executed on one computer or on multiple computers that are located at one site or distributed across multiple sites and interconnected by a communication network.

According to an embodiment, the processes and logic flows described in this specification can be performed by one or more programmable computers executing one or more computer programs to perform functions by operating on input data and generating output. The processes and logic flows can also be performed by, and apparatus can also be implemented as, special purpose logic circuitry, e.g., an FPGA an ASIC.

Computers suitable for the execution of a computer program include, by way of example, general or special purpose microprocessors or both, or any other kind of central processing unit. Generally, a CPU will receive instructions and data from a read-only memory or a random access memory or both. Elements of a computer are a CPU for performing or executing instructions and one or more memory devices for storing instructions and data. Generally, a computer will also include, or be operatively coupled to receive data from or transfer data to, or both, one or more mass storage devices for storing data, e.g., magnetic, magneto-optical disks, or optical disks. However, a computer need not have such devices. Moreover, a computer can be embedded in another device, e.g., a mobile telephone, a personal digital assistant (PDA), a mobile audio or video player, a game console, a Global Positioning System (GPS) receiver, or a portable storage device, e.g., a universal serial bus (USB) flash drive, to name just a few. Computer-readable media suitable for storing computer program instructions and data include all forms of non-volatile memory, media and memory devices, including by way of example semiconductor memory devices, e.g., EPROM, EEPROM, and flash memory devices; magnetic disks, e.g., internal hard disks or removable disks; magneto optical disks; and CD-ROM and DVD-ROM disks. The processor and the memory can be supplemented by, or incorporated in, special purpose logic circuitry.

To provide for interaction with a user, embodiments of the subject matter described in this specification can be implemented on a computer having a display device, e.g., a CRT (cathode ray tube) or LCD (liquid crystal display) monitor, for displaying information to the user and a keyboard and a pointing device, e.g., a mouse or a trackball, by which the user can provide input to the computer. Other kinds of devices can be used to provide for interaction with a user as well; for example, feedback provided to the user can be any form of sensory feedback, e.g., visual feedback, auditory feedback, or tactile feedback; and input from the user can be received in any form, including acoustic, speech, or tactile input. In addition, a computer can interact with a user by sending documents to and receiving documents from a device that is used by the user; for example, by sending web pages to a web browser on a user's device in response to requests received from the web browser.

In another embodiment, the subject matter described in this specification can be implemented in a computing system that includes a back-end component, e.g., as a data server, or that includes a middleware component, e.g., an application server, or that includes a front-end component, e.g., a client computer having a graphical user interface or a Web browser through which a user can interact with an implementation of the subject matter described in this specification, or any combination of one or more Such back-end, middleware, or front-end components. The components of the system can be interconnected by any form or medium of digital data communication, e.g., a communication network. Examples of communication networks include a local area network (LAN) and a wide area network (WAN), e.g., the Internet.

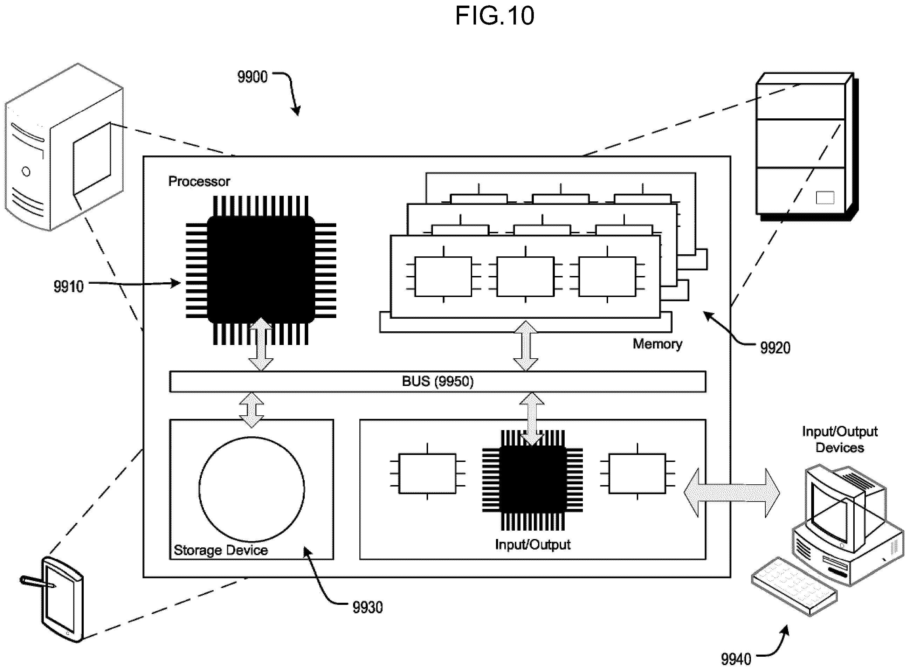

An example of a type of computer is shown in FIG. 10. The computer 9900 can be used for the operations described in association with any of the computer-implement methods described previously, according to one implementation. For example, the computer 9900 can be an example of the X-ray diagnostic apparatus 100. The computer 9900 includes processing circuitry, as discussed above. The computer 9900 can include other components not explicitly illustrated in FIG. 10, such as a CPU, GPU, frame buffer, etc. The processing circuitry includes one or more of the elements discussed next with reference to FIG. 10. In FIG. 10, the computer 9900 includes a processor 9910, a memory 9920, a storage device 9930, and an input/output device 9940. Each of the components 9910, 9920, 9930, and 9940 are interconnected using a system bus 9950. The processor 9910 is capable of processing instructions for execution within the system 9900. In one implementation, the processor 9910 is a single-threaded processor. In another implementation, the processor 9910 is a multi-threaded processor. The processor 9910 is capable of processing instructions stored in the memory 9920 or on the storage device 9930 to display graphical information for a user interface on the input/output device 9940.

The memory 9920 stores information within the computer 9900. In one implementation, the memory 9920 is a computer-readable medium. In one implementation, the memory 9920 is a volatile memory unit. In another implementation, the memory 9920 is a non-volatile memory unit.

The storage device 9930 is capable of providing mass storage for the computer 9900. In one implementation, the storage device 9930 is a computer-readable medium. In various different implementations, the storage device 9930 may be a floppy disk device, a hard disk device, an optical disk device, or a tape device.

The input/output device 9940 provides input/output operations for the computer 9900. In one implementation, the input/output device 9940 includes a keyboard and/or pointing device. In another implementation, the input/output device 9940 includes a display unit for displaying graphical user interfaces.

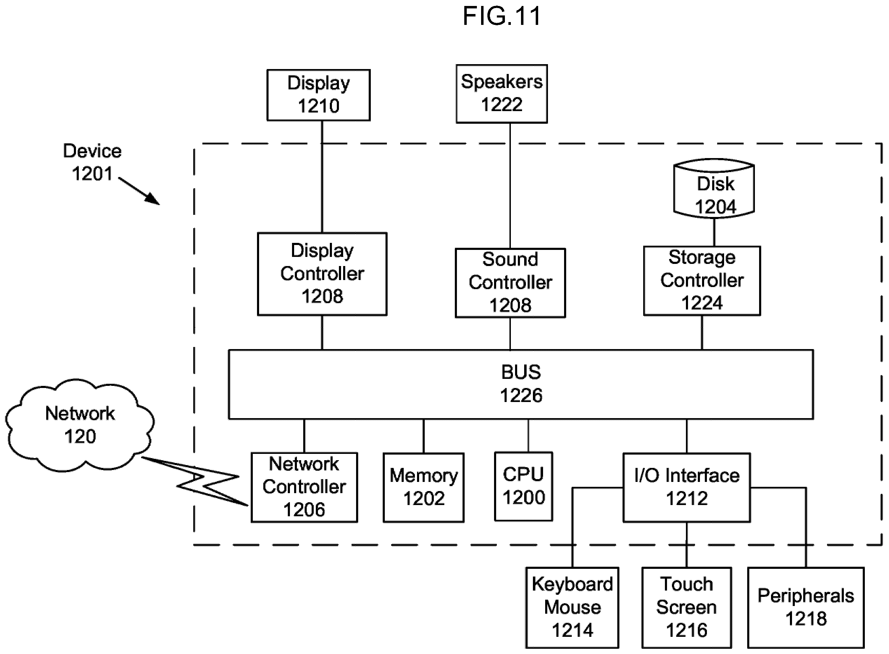

Next, a hardware description of a device according to the present embodiments is described with reference to FIG. 11. In FIG. 11, the device 1201, includes processing circuitry, as discussed above. The processing circuitry includes one or more of the elements discussed next with reference to FIG. 11. The device can include other components not explicitly illustrated in FIG. 11, such as a CPU, GPU, frame buffer, etc. In FIG. 11, the device includes a CPU 1200 which performs the processes described above/below. The process data and instructions may be stored in memory 1202. These processes and instructions may also be stored on a storage medium disk 1204 such as a hard drive (HDD) or portable storage medium or may be stored remotely. Further, the claimed advancements are not limited by the form of the computer-readable media on which the instructions of the process of the present embodiment are stored. For example, the instructions may be stored on CDs, DVDs, in FLASH memory, RAM, ROM, PROM, EPROM, EEPROM, hard disk or any other information processing device with which the device 1201 communicates, such as a server or computer.

Further, the claimed advancements may be provided as a utility application, background daemon, or component of an operating system, or combination thereof, executing in conjunction with CPU 1200 and an operating system such as Microsoft Windows, UNIX, Solaris, LINUX, Apple MAC-OS and other systems known to those skilled in the art.

The hardware elements in order to achieve the device 1201 may be realized by various circuitry elements, known to those skilled in the art. For example, CPU 1200 may be a Xenon or Core processor from Intel of America or an Opteron processor from AMD of America, or may be other processor types that would be recognized by one of ordinary skill in the art. Alternatively, the CPU 1200 may be implemented on an FPGA, ASIC, PLD or using discrete logic circuits, as one of ordinary skill in the art would recognize. Further, CPU 1200 may be implemented as multiple processors cooperatively working in parallel to perform the instructions of the processes described above.

The device in FIG. 11 also includes a network controller 1206, such as an Intel Ethernet PRO network interface card from Intel Corporation of America, for interfacing with network 120, and to communicate with the other devices. As can be appreciated, the network 120 can be a public network, such as the Internet, or a private network such as an LAN or WAN network, or any combination thereof and can also include PSTN or ISDN sub-networks. The network 120 can also be wired, such as an Ethernet network, or can be wireless such as a cellular network including EDGE, 3G, 4G and 5G wireless cellular systems. The wireless network can also be WiFi, Bluetooth, or any other wireless form of communication that is known.

The device further includes a display controller 1208, such as a NVIDIA GeForce GTX or Quadro graphics adaptor from NVIDIA Corporation of America for interfacing with display 1210, such as an LCD monitor. A general purpose I/O interface 1212 interfaces with a keyboard and/or mouse 1214 as well as a touch screen panel 1216 on or separate from display 1210. General purpose I/O interface also connects to a variety of peripherals 1218 including printers and scanners.

A sound controller 1220 is also provided in the device 1201 to interface with speakers/microphone 1222 thereby providing sounds and/or music.

The general purpose storage controller 1224 connects the storage medium disk 1204 with communication bus 1226, which may be an ISA, EISA, VESA, PCI, or similar, for interconnecting all of the components of the device. A description of the general features and functionality of the display 1210, keyboard and/or mouse 1214, as well as the display controller 1208, storage controller 1224, network controller 1206, sound controller 1220, and general purpose I/O interface 1212 is omitted herein for brevity as these features are known.

While this specification contains many specific implementation details, these should not be construed as limitations on the scope of what may be claimed, but rather as descriptions of features that may be specific to particular embodiments.

Certain features that are described in this specification in the context of separate embodiments can also be implemented in combination in a single embodiment. Conversely, various features that are described in the context of a single embodiment can also be implemented in multiple embodiments separately or in any suitable sub-combination. Moreover, although features may be described above as acting in certain combinations and even initially claimed as such, one or more features from a claimed combination can in some cases be excised from the combination, and the claimed combination may be directed to a sub-combination or variation of a sub-combination.

In the preceding description, specific details have been set forth, such as a particular geometry of a processing system and descriptions of various components and processes used therein. It should be understood, however, that techniques herein may be practiced in other embodiments that depart from these specific details, and that such details are for purposes of explanation and not limitation. Embodiments disclosed herein have been described with reference to the accompanying drawings. Similarly, for purposes of explanation, specific numbers, materials, and configurations have been set forth in order to provide a thorough understanding. Nevertheless, embodiments may be practiced without such specific details. Components having substantially the same functional constructions are denoted by like reference characters, and thus any redundant descriptions may be omitted.

According to at least one of the embodiments described above, a series of processes including X-ray image acquisition processing and image restoration processing can be made more efficient.

Some embodiments of the present invention have been described, however, these embodiments are presented as examples and are not intended to limit the scope of the invention. These embodiments can be implemented in other various forms, and various omissions, substitutions, and modifications can be made without departing from the scope of the invention as defined by the appended claims. These embodiments and variations thereof are included within the scope of the claims as well as within the scope of the invention.

**Claims**

1. An X-ray diagnostic apparatus (100) of an embodiment comprising:

   a setting unit (212) that inputs an X-ray image acquired using a first set of exposure parameters into a first model to obtain a second set of exposure parameters;
   an acquisition unit (211) that inputs the X-ray image acquired using the second set of exposure parameters into a second model trained together with the first model to obtain a restored image; and
   a control unit (213) that outputs the restored image.

2. The X-ray diagnosis apparatus of claim 1, wherein the setting unit (212) inputs a plurality of X-ray images acquired using the first set of exposure parameters into the first model to obtain the second set of exposure parameters.

3. The X-ray diagnosis apparatus of claim 2, wherein the setting unit (212) inputs a plurality of adjacent frames of X-ray images acquired using the first set of exposure parameters into the first model to obtain the second set of exposure parameters.

4. The X-ray diagnosis apparatus of claim 1, wherein, in the training process, the first model is trained to receive a first training image and output a set of training exposure parameters, the second model is trained to receive a second training image acquired based on the set of training exposure parameters and output a training restored image, and the first model is updated based on a comprehensive evaluation of an X-ray radiation dose corresponding to the set of training exposure parameters and image quality of the training restored image.

5. The X-ray diagnosis apparatus of claim 1, wherein

   the setting unit (212) obtains a sequence of a plurality of X-ray images including a first X-ray image and a second X-ray image, the first and second X-ray images being acquired sequentially using the first set of exposure parameters of an image scanning apparatus, inputs the obtained first and second X-ray images into the trained first model to obtain the second set of exposure parameters, which is output from the first model,

the acquisition unit (211) obtains a third X-ray image that was acquired by the image scanning apparatus using the second set of exposure parameters, inputs at least the second X-ray image and the third X-ray image into the trained second model to obtain a restored third X-ray image as the restored image, which is output from the second model, and

the control unit (213) outputs the restored third X-ray image as the restored image,

the first model and the second model were trained together using a training sequence of X-ray images in a training process, and

during the training process, inputs to the second model are outputs of the first model and the outputs of second model are a sequence of restored X-ray images.

6. The X-ray diagnosis apparatus of claim 5, wherein, in the training process, the first model is trained using a first loss function having a first term representing a value relating to total dosage used in acquiring the training sequence of X-ray images and a second term representing a value relating to total error in the sequence of restored X-ray images, and the second model is trained using a second loss function having a term representing the total error in the sequence of restored X-ray images.

7. The X-ray diagnosis apparatus of claim 5, wherein the first set of exposure parameters in the exposure parameters includes a first dose level and the second set of exposure parameters in the exposure parameters includes a second dose level, the first dose level being lower than the second dose level.

8. The X-ray diagnosis apparatus of claim 5, wherein the first model and the second model are alternatively trained during the training process.

9. An X-ray diagnosis apparatus, comprising: processing unit (213) configured to perform a training process by obtaining a sequence of training X-ray images sequentially acquired using a corresponding set of exposure parameters of an image scanning apparatus, inputting a pair of X-ray images of the sequence of training X-ray images into a first model to obtain an output set of exposure parameters, which is output from the first model, generating a simulation image based on the output set of exposure parameters, inputting the simulation image into a second model to obtain a restored X-ray image, which is output from the second model, comparing the restored X-ray image to a corresponding target X-ray image, of an obtained sequence of target X-ray images, to generate an image error term, repeating the inputting, generating, inputting, and comparing steps for sequential pairs of the obtained sequence of training X-ray images, and updating one of the first model and the second model.

10. The X-ray diagnosis apparatus of claim 9, wherein the processing unit (213) further performs the training process by updating the first model using a first loss function having a first term representing a value relating to total dosage used in acquiring the training sequence of X-ray images and a second term representing a total of the image error terms for the sequence of restored X-ray images, and updating the second model using a second loss function having a term representing the total of the image error terms for the sequence of restored X-ray images.

11. The X-ray diagnosis apparatus of claim 10, wherein, in the updating, the processing unit (213) is further configured to: update the first model using the first loss function while keeping the second model fixed, and update the second model using the second loss function while keeping the first model fixed.

12. The X-ray diagnosis apparatus of claim 9, wherein, in the updating, the processing unit (213) further repeats the training process for another sequence of training X-ray images sequentially acquired using another corresponding set of exposure parameters and another sequence of target X-ray images.

13. The X-ray diagnosis apparatus of claim 9, wherein the step of inputting the generated simulation image into the second model further comprises inputting another simulation image into the second model together with the simulation image, the another simulation image being generated based on another set of exposure parameters.

14. An method comprising:

inputting an X-ray image acquired using a first set of exposure parameters into a first model to obtain a second set of exposure parameters;

inputting the X-ray image acquired using the second set of exposure parameters into a second model trained together with the first model to obtain a restored image; and

outputting the restored image.

15. An method, for performing a training process, comprising:

obtaining a sequence of training X-ray images sequentially acquired using a corresponding set of exposure parameters of an image scanning apparatus;

inputting a pair of X-ray images of the sequence of training X-ray images into a first model to obtain an output set of exposure parameters, which is output from the first model;

generating a simulation image based on the output set of exposure parameters;

inputting the simulation image into a second model to obtain a restored X-ray image, which is output from the second model, comparing the restored X-ray image to a corresponding target X-ray image, of an obtained sequence of target X-ray images, to generate an image error term; and

repeating the inputting, generating, inputting, and comparing steps for sequential pairs of the obtained sequence of training X-ray images, and updating one of the first model and the second model.

FIG.1

EP 4 620 397 A1

# FIG.2

FIG.3

EP 4 620 397 A1

FIG.4

# FIG.5

## FIG.6A

## FIG.6B

# FIG.7A

## 700

```
┌─────────────────────────────────────┐
│ Obtain first image at first exposure │ ◄──┐
│              setting                  │    │
│                705                    │    │
└─────────────────────────────────────┘    │
              │                              │
              ▼                              │
┌─────────────────────────────────────┐    │
│ Determine first parameters of object │    │
│      of interest in first image      │    │
│                710                    │    │
└─────────────────────────────────────┘    │
              │                              │
              ▼                              │
┌─────────────────────────────────────┐    │
│    Obtain second image at first      │    │
│          exposure setting            │    │
│                715                    │    │
└─────────────────────────────────────┘    │
              │                              │
              ▼                              │
┌─────────────────────────────────────┐    │
│ Determine second parameters of object│    │
│     of interest in second image      │    │
│                720                    │    │
└─────────────────────────────────────┘    │
              │                              │
              ▼                              │
┌─────────────────────────────────────┐    │
│  Determine difference between first  │    │
│ parameters and second parameters of  │    │
│           object of interest         │    │
│                725                    │    │
└─────────────────────────────────────┘    │
              │                              │
              ▼                              │
          ╱───────────╲                     │
         ╱  Difference  ╲     NO             │
        ╱ greater than   ╲───────────────────┘
        ╲ threshold?     ╱
         ╲    730       ╱
          ╲───────────╱
              │ YES
              ▼
┌─────────────────────────────────────┐
│   Determine second exposure setting  │
│                735                    │
└─────────────────────────────────────┘
              │
              ▼
┌─────────────────────────────────────┐
│  Obtain third image at second        │
│          exposure setting            │
│                740                    │
└─────────────────────────────────────┘
              │
              ▼
           ( 701 )
```

# FIG.7B

## 701

700

Obtain third image at second exposure
setting
740

Determine third parameters of object of
interest in third image
745

Obtain fourth image at second exposure
setting
750

Determine fourth parameters of object of
interest in third image
755

Determine difference between third
parameters and fourth parameters of
object of interest
760

Difference
greater than threshold?
765

YES

NO

Obtain fifth image at first exposure setting
770

# FIG.7C

## 702

Obtain a sequence of training images sequentially acquired using a corresponding set of exposure parameters of an image scanning apparatus
775

Input inputting a pair of images of the sequence of training images into a first model to obtain an output set of exposure parameters, which is output from the first model
780

Generate a simulated image based on the output set of exposure parameters
785

Compare the restored image to a corresponding target image, of an obtained sequence of target images, to generate an image error term
790

Repeat the inputting, generating, inputting, and comparing steps for sequential pairs of the obtained sequence of training images
795

Update one of the first model and the second model, wherein the first model is updated using a first loss function having a first term representing a total dosage used in acquiring the training sequence of images and a second term representing a total of the image error terms for the sequence of restored images, and the second model is updated using a second loss function having a term representing the total of the image error terms for the sequence of restored images
797

# FIG.7D

## 703

Obtain a sequence of a plurality of images including a first image and a second image, the first and second images being acquired sequentially using a first set of exposure parameters of an image scanning apparatus
805

Obtain the obtained first and second images into a trained first model to obtain a second set of exposure parameters, which is output from the trained first model
810

Obtain a third image that was acquired by the image scanning apparatus using the obtained second set of exposure parameters
815

Input at least the obtained second image and the obtained third image into a trained second model to obtain a restored third image, which is output from the trained second model
820

Output the restored third image
825

FIG.8A

FIG.8B

FIG.9

X-RAY DIAGNOSTIC APPARATUS

100

DISPLAY 23

IMAGE PROCESSING CIRCUITRY 26

STORAGE 25

IMAGE DATA GENERATING CIRCUITRY 24

PROCESSING CIRCUITRY 21

ACQUISITION FUNCTION 211

SETTING FUNCTION 212

CONTROL FUNCTION 213

INPUT CIRCUITRY 22

C-ARM ROTATING/MOVING MECHANISM 17

TABLETOP MOVING MECHANISM 18

C-ARM/TABLETOP MECHANISM CONTROL CIRCUITRY 19

COLLIMATOR CONTROL CIRCUITRY 20

X-RAY DETECTOR 16

15

P

14

COLLIMATOR 13

X-RAY TUBE 12

HIGH VOLTAGE GENERATOR 11

# FIG.10

# FIG.11

Device 1201

Display 1210

Speakers 1222

Disk 1204

Display Controller 1208

Sound Controller 1208

Storage Controller 1224

BUS 1226

Network 120

Network Controller 1206

Memory 1202

CPU 1200

I/O Interface 1212

Keyboard Mouse 1214

Touch Screen 1216

Peripherals 1218

EP 4 620 397 A1

Europäisches
Patentamt

European
Patent Office

Office européen
des brevets

# EUROPEAN SEARCH REPORT

Application Number

EP 25 16 5393

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2023/038871 A1 (VAN VEEN DAVID [US] ET AL) 9 February 2023 (2023-02-09)<br>* abstract *<br>* paragraph [0004] *<br>* paragraph [0007] *<br>* paragraph [0034] - paragraph [0035] *<br>* paragraph [0059] - paragraph [0067] *<br>* figure 3 *<br>----- | 1-15 | INV.<br>A61B6/00<br>A61B6/58 |
| A | US 2022/414832 A1 (HU YI [US] ET AL) 29 December 2022 (2022-12-29)<br>* abstract *<br>* paragraph [0001] *<br>* paragraph [0003] *<br>* paragraph [0046] - paragraph [0047] *<br>* paragraph [0054] *<br>* paragraph [0060] - paragraph [0061] *<br>* figure 2 *<br>----- | 1-15 | |
| A | WO 2024/008721 A1 (KONINKLIJKE PHILIPS NV [NL]) 11 January 2024 (2024-01-11)<br>* abstract *<br>* paragraph [0015] - paragraph [0022] *<br>* paragraph [0044] *<br>* claim 1 *<br>----- | 1-15 | TECHNICAL FIELDS SEARCHED (IPC)<br><br>A61B<br>G06T |

The present search report has been drawn up for all claims

| Place of search | Date of completion of the search | Examiner |
|---|---|---|
| Munich | 29 July 2025 | Montes, Pau |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 16 5393

This annex lists the patent family members relating to the patent documents cited in the above-mentioned European search report.
The members are as contained in the European Patent Office EDP file on
The European Patent Office is in no way liable for these particulars which are merely given for the purpose of information.

29-07-2025

| Patent document cited in search report | Publication date | Patent family member(s) | | Publication date |
|---|---|---|---|---|
| US 2023038871 A1 | 09-02-2023 | CN | 115397332 A | 25-11-2022 |
| | | EP | 4103061 A1 | 21-12-2022 |
| | | US | 2023038871 A1 | 09-02-2023 |
| | | WO | 2021163022 A1 | 19-08-2021 |
| US 2022414832 A1 | 29-12-2022 | EP | 4123572 A2 | 25-01-2023 |
| | | JP | 2023004941 A | 17-01-2023 |
| | | US | 2022414832 A1 | 29-12-2022 |
| WO 2024008721 A1 | 11-01-2024 | CN | 119522440 A | 25-02-2025 |
| | | EP | 4552070 A1 | 14-05-2025 |
| | | JP | 2025520779 A | 03-07-2025 |
| | | WO | 2024008721 A1 | 11-01-2024 |

EPO FORM P0459

For more details about this annex : see Official Journal of the European Patent Office, No. 12/82